# EUROPEAN PATENT APPLICATION

(11) **EP 2 530 615 A1**
(43) Date of publication of application: **05.12.2012**
(21) Application number: 11004471.6
(22) Date of filing: 01.06.2011
(51) Int. Cl.: G06F 19/12

(54) **Method for modelling, optimizing, parameterizing, testing and/or validating a dynamic network or network perturbations**

(71) Applicant: Albert-Ludwigs-Universität Freiburg, 79085 Freiburg (DE)
(72) Inventor: Thedieck, Kathrin, 79541 Lörrach (DE); Sonntag, Annika, 79098 Freiburg (DE); Shanley, Daryl, Gateshead NE 9 6DH (GB); Dalle Pezze, Piero, NE1 5PG Newcastle upon Tyne (GB)
(74) Representative: Langfinger, Klaus Dieter

(57) **Abstract**

A method (2) for modelling, optimizing, parameterizing, testing and/or validating a dynamic network or network perturbations, for predicting the response of a structure (4), of a group of structures (16) and/or of a network profile (18), in particular of a common or individual network profile, as a result to a perturbation, in particular to an agent (6) or a combination of agents (8), e.g. a kinase inhibitor/activator, and/or for predicting of effects of specific profiles, in particular mutational or metabolic or inhibitor/activator profiles on system behaviour, in particular cellular growth or clinical outcome.

## Description

The invention relates to a method for modelling, optimizing, parameterizing, testing and/or validating a dynamic network or network perturbations, for predicting the response of a structure, of a group of structures and/or of a network profile, in particular of a common or individual network profile(s) as a result of a perturbation, in particular in response to an agent or a combination of agents, e.g. a kinase inhibitor/activator, and/or for predicting effects of specific profiles, in particular mutational or metabolic or inhibitor/activator profiles on system behaviour, in particular cellular growth or clinical outcome and to a use of said method.

Methods of this type are principally known, in particular methods directly related to signalling.

I.e. models for static networks are known that require careful inclusion of known molecular interactions reported in the literature or in databases.

Models for dynamic networks additionally require detailed quantitative experimental time course data.

Many models rely on published data from external sources. But to reach the most suitable readouts and to reach ensuring coherence of the data sets the data for the known models should be generated specifically for the model.

Since most combined experimental-computational approaches address very specific questions the generated data sets are often of narrow scope, covering one single or two time points or comparatively limited parts of the network. In particular, none of the existing models, in particular models of Insulin - mammalian Target Of Rapamycin kinase (mTOR) signalling integrates mammalian Target Of Rapamycin Complex 2 (mTORC2) regulation.

Therefore, the known methods need to be calibrated for every specific question which is time-consuming and expensive.

Accordingly, it was an object of the instant invention to suggest a method that extends usability for different specific questions and that improves prediction of the response of a structure in reaction to a perturbation, in particular to an agent or a combination of agents.

This object is achieved with a method in accordance with claim 1. Preferred embodiments are set forth in the dependent claims.

The instant invention provides a method for modelling, optimizing, parameterizing, testing and/or validating a dynamic network or network perturbations, for predicting the response of a structure, of a group of structures and/or of a network profile, in particular a common or individual network profile as a result of a perturbation, in particular in response to an agent or a combination of agents, e.g. a kinase inhibitor/activator, and/or for predicting effects of specific profiles, in particular mutational or metabolic or inhibitor/activator profiles on system behaviour, in particular cellular growth or clinical outcome comprising the steps:
a. selecting at least one appropriate structure model or specific profile model of the at least one structure, of the group of structures and/or of the network profile;
b. identifying at least one perturbation, in particular at least one agent and/or at least one combination of agents and, if necessary, identifying the concentration of each perturbation, in particular agent or combination of agents;
c. parameterizing of the at least one structure model or specific profile model of the at least one structure profile and/or of the at least one combination of structure models or specific profile models, whereby the structure model or specific profile model and/or the combination of structure models or specific profile models uses at least a dynamic network model, in particular of insulin-(mTOR) kinase model for signalling or regulation;
d. reducing the number of parameters, in particular the parameters for parameterizing the at least one structure model or specific profile model and/or the at least one combination of structure models or specific profile models of the structure, in particular the dynamic network model, to generate a reduced structure, in particular a reduced dynamic network model;
e. calculating the response of each structure, of the group of structures and/or of the common pathway profile caused by a perturbation, in particular an agent and/or combination of agents;
f. defining at least one subcollective of structure profiles, of a group of structures and/or of the network profile characterized by the response with the best outcome;
g. displaying at least the profile subcollective, the corresponding agent and/or the combination of agents.

The step of parameterization can also comprise everything within the pathway, i.e. proteins, involved mutations and/or the like.

Additionally the sequence of the steps of the method can vary from the above described sequence.

Generally the structure, the group of structures and/or the network profile can be found in any suitable way. However, it has proven to be advantageous that the method comprises a step of identifying at least one structure, one group of structures and/or a network profile, in particular by uploading from a database and/or by experimental determination.

The inventive method displays the effect of perturbations, in particular of an agent or a combination of agents, and allows a prediction of the best selection of perturbations, in particular agents or combinations of agents to reach the best result on structures or groups of structures.

Furthermore, it can be advantageous, if the reduction of the parameter number, in particular for parameterizing the at least one structure profile and/or the at least one combination of structure profiles is iterative, whereby in each step the value of all undetermined parameters is optimized multiple times. Additionally, it is also possible that at least one parameter is reducible by manual setting and/or database related setting.

Generally, the iteration within the step of parameter reduction can be set manually or automatically. I.e. the iteration can be infinite or limited. In particular, if the iteration is limited, the total number of iterations can be set manually or automatically.

It is preferred that the parameters that cluster within the set of best solution are assigned values and/or that the reduction is repeated with the remaining undetermined parameters until all parameters are assigned a value.

The best solution can be defined in several manners. According to one embodiment, it can prove to be advantageous to define the best solution by giving minimal difference between the dynamic network and given data. Therefore the number of iterations is related to a fault tolerance.

For improving the step of reducing it has proved advantageous in certain cases that boundaries of reducing the dynamic network model and/or of a level of, in particular molecular, detail can be set manually and/or automatically.

Moreover, in another embodiment of the invention the parameterization and/or the reduction comprises an initial estimation for at least one parameter, in particular by random generation within the boundaries and in particular for a certain number of initial receptor molecules amount and the related rate constants, in particular in the preferred embodiment at least three receptor initial molecules amounts and three kinetic rates constants in case of the mTOR model.

The parameters of the reduced dynamic model can be selected and/or chosen at will. However, it has proven to be advantageous if the reduced dynamic model comprises parameters of interactions with dynamic behavior such as feedback mechanisms and/or comprises parameters of molecules and interactions that can be measured.

Moreover, in a further embodiment of the invention the reduced dynamic model comprises at least three receptor initial molecules amounts and three kinetic rates constants.

The parameterization may generally comprise any data. However, it has proven to be advantageous if parameterization comprises dynamic quantitative time course data using a reuse-orientated calibration process to introduce several different network structures without recalibration.

The perturbation, in particular the agent or the group of agents, can target anything that inhibits or activates a response of the structure or the group of structures. According to a preferred embodiment, the perturbation, the agent or the combination of agents targets mTOR, in particular in one or several mTOR complexes, in particular mTOR complex 1 (mTORC1) and/or mTORC2, entirely or partly, directly or indirectly.

According to a preferred embodiment mTOR complexes, in particular their activity, in particular the activity of mTORC1 and/or mTORC2 are calculated by the dynamic network model, in particular the reduced dynamic network model, in particular based on dynamic, in particular quantitive time course model output.

The time course model output may be validated in any way. However, it has proven to be advantageous if the dynamic time course model output is validated experimentally.

To increase the quality of the output it has proven to be advantageous if parameterization comprises a structure component modification, in particular molecule modification, in particular protein or lipid modification, in particular with the steps of:
a. calibration of the dynamic network model by assuming a signal input, in particular receptor, in particular insulin receptor network dependent regulation;
b. calibration of the network model by assuming the network model independent of mTORC2 and of phospho-inositide-dependent protein kinase 2 (PDK2), in particular by assuming that dynamics are regulated by autoactivation;
c. calibration of the network model by assuming the dynamic network model dependent on mTORC2;
d. calibration of the network model by assuming the network model dependent on PDK2.

In a further embodiment of the inventive method the steps of calibration of the dynamic network model comprise at least one hypothesis for mTORC2 activation that substitutes the mTORC2 dynamics, in particular by:
a. recalibration of the network model by assuming the model as phosphatidylinositol-3-kinase (PI3K) - independent,
b. recalibration the network model by assuming the model as PI3K-dependent,
c. recalibration the network model by assuming the model tuberous sclerosis complex 1/2 (TSC1/TSC2) complex-independent or
d. recalibration the network model by assuming the model TSC1/TSC2 complex-dependent.

The parameterization can comprise a step of calibration or can be realized without calibration. It has proven to be advantageous if parameterization comprises a calibration having the calibration steps:
a. selecting parameters to calibrate;
b. initial estimation for at least one parameter to be optimized, in particular from initial configuration;
c. selecting the best solution;
d. fixing of common parameters;
e. selecting the next unfixed parameters and return to step b or end calibration.

The response of a structure to a treatment can comprise any reaction of the structure. In a preferred embodiment the response to a treatment comprises the response of tumor cells, in particular kinase acticity, in particular mTOR activity, in particular cellular growth.

Additionally, it has proven to be advantageous if a set of gradual in silico and experimental perturbations are established.

Furthermore, in one embodiment of the method the parameterization of each common mutational profile of the at least one cell and/or of the group of cells is based on quantitative measurements.

Furthermore, in one preferred embodiment of the invention the dynamic network comprises a dynamic mTOR network or network model of insulin-mTOR kinase signalling or regulation. According to another preferred embodiment the structure comprises a, in particular biological, cell, e.g. a tumor cell. In still another preferred embodiment of the invention the agent or combination of agents comprises a kinase inhibitor/activator. It is to be understood that all these preferred embodiments may be combined in any manner with each other

Another embodiment of the instant invention relates to the use of the method of the instant invention for predicting the effect of the response of a structure in reaction to a perturbation, in particular in response to an agent or a combination of agents, e.g. a kinase inhibitor/activator, and/or for predicting of effects of specific profiles, in particular mutational or metabolic or inhibitor/activator profiles on system behaviour, in particular cellular growth or clinical outcome inhibiting/activating agents, in particular in combination or different concentrations, on clinical outcome in response to, in particular inhibiting and/or activating, agents or combinations of agents having at least one of the previous features.

Furthermore, according to a preferred embodiment such use comprises
a. subgrouping of patient cohorts according to mutational profiles;
b. selecting of patient cohorts comprising mutational profiles or tumor types with good clinical prognosis in response to perturbations, agents or combinations of agents, in particular combinations of inhibitor(s), and;
c. reduce patient cohorts for proof of efficacy in clinical studies.

The method and the use of the method are advantageous in many ways.

First, the method can be used for several different network structures without recalibration. Therefore, the range of use of the method is extended compared to existing systems. Furthermore, the method allows identification of patient populations with mutational patterns presenting a high probability of positive response perturbations, in particular to pharmaceutically active products or combinations thereof.

Additionally, identification of perturbations, in particular of agents or agent combinations, especially preferred of kinase inhibitor/activator combinations and concentrations with the most beneficial clinical effect in patient subpanels with differential mutational or metabolic profiles is possible.

A further benefit is the reduction of patient cohorts, which improves efficacy and statistical strength in clinical studies.

Finally, an improvement of clinical outcomes by appropriate patient selection is enabled.

Additional features, details and advantages of the invention are given in the accompanying claims and in the technical drawings and subsequent description of one embodiment of the invention.

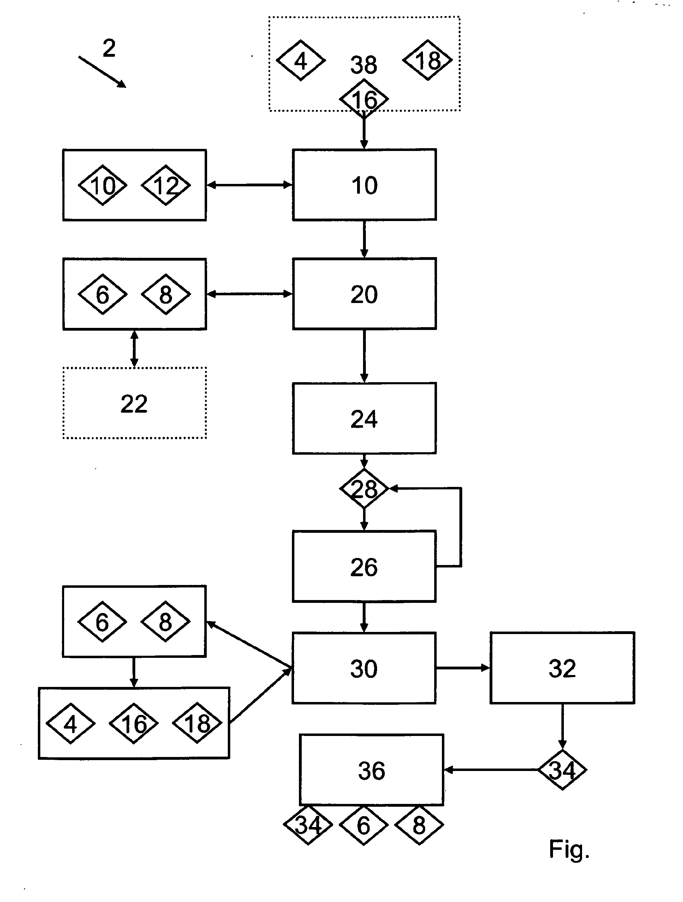

The drawing shows:
Figure a schematic view of a first embodiment of the model;

The Figure shows a schematic view of a first embodiment of a method 2 for modelling, optimizing, parameterizing, testing and/or validating a dynamic network or network perturbations, for predicting the response of a structure 4 as a result of a perturbation, in particular to an agent 6 or a combination of agents 8, e.g. a kinase inhibitor/activator, and/or for predicting of effects of specific profiles, in particular mutational or metabolic or inhibitor/activator profiles on system behaviour, in particular cellular growth or clinical outcome.

The method 2 comprises a step of selecting 10 at least one appropriate structure model 12 or specific profile model 14 of the at least one structure 4, of a group of structures 16 and/or of the network profile 18.

Subsequently, a step of identifying 20 of the at least one agent 6 and/or at least one combination of agents 8 is realised. If it is necessary a step of identifying 22 the concentration of each agent 6 or of each combination of agents 8 can be applied.

After identifying 20 of the at least one agent 6 and/or the at least one combination of agents 8 a step of parameterization 24 of the at least one structure profile and/or of at least one combination of structure profiles is carried out, whereby the structure profile and/or the combination of structure profiles use at least a dynamic network model, in particular an insulin-TOR kinase model;

In a following step 26 the number of parameters 28, in particular the parameters 28 defined for 24 the at least one structure profile and/or the at least one combination of structure profiles of the dynamic network model to generate a reduced dynamic network model, is reduced;

After that a step of calculating 30 the reaction of each structure 4, of the group of structures 18 and/or of the network profile 20 caused by the agent 6 and/or combination of agents 8 is carried out.

Dependent from the calculated reaction of each structure 4, of the group of structures 18 and/or of the network profile 20 the next step 32 defines at least one subcollective 34 of pathway profiles of the structure, of the group of structures and/or of a common network profile characterised by reaction with the best outcome;

Finally the method 2 provides a step of displaying 36 at least the subcollective 34, the corresponding agent 6 and/or the combination of agents 8.

Prior to the step 10 of selecting ,a step of identifying 38 at least one structure 4, one group of structures 16 and/or one network profile 18 can be carried out if necessary or advantageous, in particular by uploading from a database and/or by experimental determination.

The features of the present invention disclosed in the description above, in the claims and in the drawings can be used for implementing the invention in its different embodiments both individually and in every possible combination thereof.

### REFERENCE NUMERALS

- 2: Method
- 4: Structure
- 6: Agent
- 8: Combination of agents
- 10: Step of selecting
- 12: Appropriate structure model
- 14: Specific profile
- 16: Group of structures
- 18: Common pathway profile
- 20: Step of identifying agent
- 22: Step of identifying concentration of agent
- 24: Step of parameterizing at least the agent
- 26: Step of reducing
- 28: Parameters
- 30: Step of calculating
- 32: Step of defining
- 34: Subcollective
- 36: Step of displaying
- 38: Step of identifying

## Claims

1. A method (2) for modelling, optimizing, parameterizing, testing and/or validating a dynamic network or network perturbations, for predicting the response of a structure (4), of a group of structures (16) and/or of a network profile (18), in particular of a common or individual network profile, as a result to a perturbation, in particular to an agent (6) or a combination of agents (8), e.g. a kinase inhibitor/activator, and/or for predicting of effects of specific profiles, in particular mutational or metabolic or inhibitor/activator profiles on system behaviour, in particular cellular growth or clinical outcome, comprising the steps:
a. selecting (10) at least one appropriate structure model (12) or specific profile model (14) of the at least one structure (4), of the group of structures (16) and/or of the network profile (18);
b. identifying (20) at least perturbation, in particular at least one agent (6) and/or at least one combination of agents (8) and, if necessary, identifying (22) the concentration of each perturbation, agent or combination of agents (6, 8);
c. parameterizing (24) of at least one structure model or specific profile model of the at least one structure profile of the at least one combination of structure models or specific profile models, whereby the structure models or specific profile models use at least a dynamic network model, in particular of insulin-mTOR kinase model for signalling or regulation;
d. reducing (26) the number of parameters (28), in particular the parameters (28) for parameterizing (24) the at least one structure model or specific profile model and/or the at least one combination of structure models or specific profile models of the structure, in particular of a dynamic network model to generate a reducedstructure, in particular a reduced dynamic network model;
e. calculating (30) the response of each structure (4), of the group of structures (16) and/or of the network profile (18) caused by the perturbation, in particular by an agent (6) and/or combination of agents (8) ;
f. defining (32) at least one subcollective (34) of structure profiles, of the group of structures (16) and/or of the network profile (18) caused by the response with the best outcome;
g. displaying (36) at least the profile subcollective (34), the corresponding perturbation, in particular the corresponding agent (6) and/or the combination of agents (8).

2. Method (2) according to claim 1, **characterized in that** the reduction (26) of the number of parameters (28), in particular the parameters (28) generated by parameterizing (24) the at least one structure model or specific profile model is iterative, whereby in each step the value of all undetermined parameters is optimized multiple times.

3. Method (2) according to claim 2, **characterized in that** the parameters (28) that cluster within the set of best solution are assigned values and/or that the reduction (26) is repeated with the remaining undetermined parameters (28) until all parameters (28) are assigned a value.

4. Method (2) according to one or more of the foregoing claims, **characterized in, that** boundaries of reducing (26) the dynamic network model and/or of a level of, in particular molecular, detail can be set manually and/or automatically.

5. Method (2) according to one or more of the foregoing claims, **characterised in that** the parameterizing (24) and/or reducing (26) comprises an initial estimation for at least one parameter (28), in particular by random generation within the boundaries and in particular for at certain number of initial receptor molecules amount and the related rate constants, in particular of at least three receptor initial molecules amounts and three kinetic rates constants for a mTOR model.

6. Method (2) according to one or more of the foregoing claims, **characterized in that** the reduced dynamic model comprises parameters (28) of interactions with dynamic behavior such as feedback mechanisms and/or comprises parameters of molecules and interactions that can be measured.

7. Method (2) according to one or more of the foregoing claims, **characterized in that** parameterization (24) comprises dynamic quantitative time course data using a reuse-orientated calibration process to introduce several different network structures without recalibration.

8. Method (2) according to one or more of the foregoing claims, **characterized in that** the perturbation, in particular the agent (6) or the combination of agents (8) targets mTOR, in particular mTORC1 and/or mTORC2, entirely or partly, directly or indirectly.

9. Method (2) according to one or more of the foregoing claims, **characterized in that** the dynamic time course model output is validated experimentally.

10. Method (2) according to one or more of the foregoing claims, **characterized in that** parameterizing (24) comprises a structure component modification, in particular molecule modification, in particular protein or lipid modification, in particular with the steps:
a. calibration of the dynamic network model by assuming a signal input, in particular receptor, in particular insulin an insulin receptor network dependent regulation;
b. calibration of the network model by assuming the network model independent of mTORC2 and of PDK2, in particular by assuming that dynamics are regulated by autoactivation;
c. calibration of the network model by assuming the dynamic network model dependent on mTORC2;
d. calibration of the network model by assuming the network model dependent on PDK2.

11. Method (2) according to claim 10, **characterized in that** the steps of calibration of the dynamic network model comprise at least one hypothesis for mTORC2 activation that substitutes the mTORC2 dynamics, in particular by:
a. recalibration the network model by assuming the model as PI3K-independent,
b. recalibration the network model by assuming the model as PI3K-dependent,
c. recalibration the network model by assuming the model TSC1/TSC2 complex independent or
d. recalibration the network model by assuming the model TSC1/TSC2 complex dependent.

12. Method (2) according to one or more of the foregoing claims, **characterized in, that** parameterization comprises a calibration having the calibration steps:
a. selecting parameters to calibrate;
b. initial estimation for at least one parameter to be optimized, in particular from initial configuration;
c. selecting the best solution;
d. fixing of common parameters;
e. selecting the next unfixed parameters and return to step b or end calibration.

13. Method (2) according to one or more of the foregoing claims, **characterized in, that** the dynamic network comprises a dynamic mTOR (mammalian Target Of Rapamycin) network or network model of insulin-mTOR kinase signaling or regulation, the structure (4) comprises a, in particular biological cell, e.g. a tumor cell and/or the agent (6) comprises a kinase inhibitor/activator.

14. Use of the method (2) as defined in any of claims 1 to 13 for predicting the effect of the response of a structure (4), of a group of structures (16) and/or of a network profile (18) as a result of a perturbation, in particular of an agent (6) or a combination of agents (8), e.g. a kinase inhibitor/activator, and/or for predicting of effects of specific profiles, in particular mutational or metabolic or inhibitor/activator profiles on system behaviour, in particular cellular growth or clinical outcome, inhibiting/activating agents, in particular in combination or different concentrations, on clinical outcome in response to, in particular inhibiting and/or activating, agents (6) or combinations of agents (8).

15. The use of claim 14, **characterized in, that** it comprises
a. subgrouping of patient cohorts according to mutational profiles;
b. selecting of patient cohorts comprising mutational profiles or tumor types with good clinical prognosis in response to perturbations, agents or combinations of agents, in particular inhibitor(s;
c. reduce patient cohorts for proof of efficacy in clinical studies.
